# EUROPEAN PATENT APPLICATION

(11) **EP 4 303 309 A1**
(43) Date of publication of application: **10.01.2024**
(21) Application number: 22762929.2
(22) Date of filing: 10.02.2022
(51) Int. Cl.: C12P 13/04, C12N 1/21, C12N 15/54

(54) **ERGOTHIONEINE PRODUCTION METHOD**

(30) Priority: 01.03.2021 JP 2021031642
(71) Applicant: Nagase & Co., Ltd., Osaka 550-8668 (JP)
(72) Inventor: NAKATANI, Takeshi, Kobe-shi, Hyogo 651-2241 (JP); NAKASHIMA, Nanami, Kobe-shi, Hyogo 651-2241 (JP); NOGUCHI, Yuji, Kobe-shi, Hyogo 651-2241 (JP)
(74) Representative: dompatent von Kreisler Selting Werner - Partnerschaft von Patent- und Rechtsanwälten mbB
(86) International application number: PCT/JP2022/005262
(87) International publication number: WO 2022/185872

(57) **Abstract**

With the present invention, ergothioneine or a related substance thereof is produced at a low cost in a large amount through biosynthesis. The present invention pertains to: a production method that is for ergothioneine or a related substance thereof and that comprises a step for culturing a microorganism having an increased adenosine kinase (Adk) activity; and a microorganism having an increased Adk activity and having the ability to produce ergothioneine or a related substance thereof.

## Description

### RELATED APPLICATIONS

This application claims the priority benefit of Application No. 2021-031642 filed with the Japan Patent Office on March 1, 2021. The priority application is hereby incorporated by reference in its entirety.

### TECHNICAL FIELD

The present disclosure relates to a method for producing ergothioneine (EGT) or a related substance thereof, or a mixture thereof, and a microorganism that produces ergothioneine or a related substance thereof, or a mixture thereof. Specifically, the present disclosure relates to a method for producing ergothioneine or a related substance thereof, or a mixture thereof, comprising culturing a microorganism having increased activity of adenosine kinase (Adk), and a microorganism having increased Adk activity and an ergothioneine biosynthetic gene. The present disclosure enhances the ability to produce ergothioneine or a related substance thereof by increasing the Adk activity.

### BACKGROUND ART

Ergothioneine is contained in some microorganisms, especially Basidiomycetes, and is also contained in small amounts in animals and plants. Although mammals cannot biosynthesize ergothioneine, it is suggested that ergothioneine is taken into the body by eating mushrooms such as basidiomycetes, and ergothioneine is accumulated in many internal organs and organs such as the epidermis, brain, liver, kidney, spinal cord, and eyes to protect cells. As methods for producing ergothioneine, a method of culturing and extracting microorganisms such as basidiomycetes and an organic synthesis method are known (see Patent Documents 1 and 2, etc.). However, the extraction method from Basidiomycetes or the like has not been widely used for reasons such as low productivity, a long period of time for culturing the microorganisms, a complicated production process for extracting from the cells due to accumulation in the cells, and difficulty in improvement of the microorganisms. Organic synthesis methods also have not been widely used for reasons such as complicated reactions involving multiple steps, the possible use of harmful raw materials, and high costs due to the use of expensive raw materials. In addition, a fermentation production method using a microorganism in which an ergothioneine biosynthetic gene is overexpressed, or a microorganism capable of producing ergothioneine or a related substance thereof in which histidine ammonia lyase activity is reduced or eliminated, or histidine ammonia lyase gene expression is reduced or eliminated has been developed, but a more efficient method for producing ergothioneine is desired (Patent Document 3).

### PRIOR ART DOCUMENTS

### PATENT DOCUMENTS

Patent Document 1: JP-A-2006-160748
Patent Document 2: JP-A-2007-300916
Patent Document 3: WO2017/150304

### SUMMARY OF THE INVENTION

### PROBLEMS TO BE SOLVED BY THE INVENTION

As described above, there is a problem with the supply of ergothioneine or a related substance thereof. Therefore, there is a demand for a method for producing ergothioneine or a related substance thereof at low cost and in a large amount.

### SOLUTIONS TO THE PROBLEMS

As a result of intensive research to solve the above-mentioned problem, the present inventors surprisingly found that the amount of ergothioneine produced is increased by using a microorganism in which the Adk activity is increased, and have completed the present invention.

That is, the present disclosure relates to a method for producing ergothioneine or a related substance thereof, or a mixture thereof, comprising: culturing in a medium a microorganism having an ergothioneine biosynthetic gene, and collecting ergothioneine or a related substance thereof, or a mixture thereof from the medium obtained by the culture or the bacterial cells, in which the microorganism is a strain modified to increase adenosine kinase (Adk) activity. The present disclosure also relates to a microorganism having an ergothioneine biosynthetic gene, in which the microorganism has an increased Adk activity. That is, the present disclosure relates to enhancing the ability to produce ergothioneine or a related substance thereof by increasing the Adk activity.

Accordingly, the present disclosure provides:
[1] A method for producing ergothioneine or a related substance thereof, or a mixture thereof, comprising:
   culturing in a medium a microorganism having an ergothioneine biosynthetic gene, and collecting ergothioneine or a related substance thereof, or a mixture thereof from the medium obtained by the culture or the bacterial cells,
   in which the microorganism is a strain modified to increase adenosine kinase (Adk) activity;
[2] The method of [1], in which the Adk activity is increased by enhancing an expression of a gene encoding Adk;
[3] The method of [1] or [2], in which the microorganism belongs to the family *Enterobacteriaceae* or to the order *Actinomycetes;*
[4] A microorganism having an ergothioneine biosynthetic gene, in which the microorganism having an increased Adk activity;
[5] The microorganism of [4], in which the Adk activity is increased by enhancing an expression of a gene encoding Adk;
[6] The microorganism of [4] or [5], in which the microorganism belongs to the family *Enterobacteriaceae* or to the order *Actinomycetes.*

### EFFECTS OF THE INVENTION

The present disclosure provides ergothioneine or a related substance thereof inexpensively and in a large amount.

### BRIEF DESCRIPTION OF THE DRAWINGS

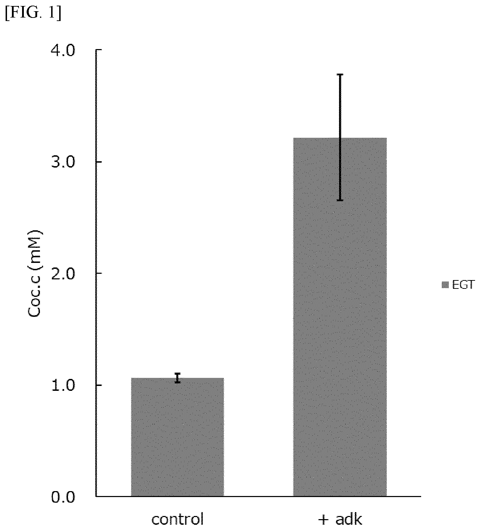

Figure 1 is a graph showing the total amount of extracellular ergothioneine produced after each of EGT-producing strains BW25113/pACG-EGT (control) and BW25113/pACG-EGT_*adk* (+*adk*) was cultured for 72 hours.

### DETAILED DESCRIPTION

In a first aspect, the present disclosure is a method for producing ergothioneine or a related substance thereof, or a mixture thereof, comprising: culturing in a medium a microorganism having an ergothioneine biosynthetic gene, and collecting ergothioneine or a related substance thereof, or a mixture thereof from the medium obtained by the culture or the bacterial cells, in which the microorganism is a strain modified to increase adenosine kinase (Adk) activity. According to the present disclosure, the amount of the production of ergothioneine or a related substance thereof can be dramatically increased by increasing the activity of Adk in a microorganism.

Ergothioneine-related substance includes ergothioneine in an oxidized form, an alkylated form. Specific examples of the oxidized form include ergothioneine sulfenic acid, ergothioneine sulfinic acid, ergothioneine sulfonic acid, a disulfide conjugate form, a mixed disulfide form, and Hercynine. Examples of the alkylated form include S-Methyl-Ergothioneine. It has been reported that the oxidized or alkylated form of ergothioneine is produced from ergothioneine through an intracellular metabolic reaction or an oxidation reaction with oxygen or an oxidizing agent in the culture medium (L. Servillo et al., Free. Radic. Biol. Med., 2015, 79, 228-36, and R. M. Y. Tang, et al., Sci. Rep., 2018, 8(1), 1601). In addition, dimethylthiohistidine and selenoneine are also exemplified as a related substance of ergothioneine. A related substance of ergothioneine also includes, but is not limited to, an ergothioneine precursor such as monomethylhistidine, dimethylhistidine, or trimethylhistidine.

"Protein activity is increased" means that the activity of the protein is higher than that of the parent strain. "Increased activity of a protein" specifically means that the activity of the protein per cell is higher than that of the parent strain. "Parental strain" means a control strain that has not been engineered to increase the activity of the target protein. The parental strain includes a wild strain and a type strain. More specifically, it can mean that the number of molecules of the protein per cell is increased and/or the function per molecule of the protein is increased compared to the parental strain. In addition to modulating the catalytic activity of a protein, the activity of the protein can be increased by modulating the amount of transcription or translation of a gene encoding the protein. In addition, "the activity of the protein is increased" includes not only increasing the activity of the target protein in a microorganism that originally has the activity of the target protein, but also conferring the activity of the protein in a microorganism that does not originally have the activity of the target protein. Furthermore, protein activity may be conferred by increasing or decreasing a function of a transcription factor such as a repressor and an activator, that a host originally has, as long as the activity of the protein is increased as a result.

The degree of increase in a protein activity is not particularly limited as long as the protein activity is increased compared to the parent strain. The activity of the protein is preferably increased by, for example, 1.5-fold or more, 2-fold or more, 3-fold or more, 4-fold or more, 5-fold or more, 6-fold or more, 7-fold or more, 8-fold or more, 9-fold or more, or 10-fold or more than that of the parent strain. When the parent strain does not have the activity of the target protein, the protein can be produced by introducing a gene encoding the protein. For example, the protein is preferably produced to the extent that the activity of the protein can be measured or more. The increase in the protein content can be confirmed by a known method such as Western blotting or by measuring the activity of the protein.

A method for increasing a protein activity and a method for increasing a gene expression of a protein are also known, for example, but are not limited to, a method using homologous recombination, mutagenesis, genome editing, or a method for removing a substance that inhibits the protein. In addition, the microorganism used in the present disclosure may have an increased activity of the protein as a result of natural mutation.

The activity of a protein can be increased by, for example, increasing the expression of a gene encoding the protein. "Enhanced gene expression" means that the expression level of the gene is higher than that of the parent strain. It is preferable that the expression level of the gene is increased by 1.5-fold or more, 2-fold or more, 3-fold or more, 4-fold or more, 5-fold or more, 6-fold or more, 7-fold or more, 8-fold or more, 9-fold or more, or 10-fold or more compared to the parent strain. When the parent strain does not have the activity of the target protein, the gene encoding the protein may be introduced or expressed to the extent that the expression level of the gene can be confirmed or more, but the gene is preferably introduced or expressed to the extent that the activity of the protein can be measured or more. Quantification of gene expression can be performed by a known method such as Northern blotting and real-time PCR.

Gene expression can be enhanced using a known method for enhancing gene expression. In general, a gene is placed under the control of a promoter having strong expression. For example, gene expression may be enhanced by introducing into a microorganism an expression vector into which a promoter and gene suitable for gene expression is incorporated, or by integrating the gene into the genome of a host by homologous recombination or the like. A plasmid vector or a virus vector can be used as a vector. When a vector is constructed, a component of the vector such as a promoter, a terminator, and a marker gene such as a drug resistance gene and a metabolic enzyme gene can be appropriately selected and used. A method for introducing a gene into a microorganism is also known. A gene to be introduced can be introduced into a microorganism as it is or by incorporating it into a vector. Examples of a gene introduction method include a lipofection method, a calcium phosphate method, a method using a polymer, an electroporation method, a particle gun method. These methods can be appropriately selected depending on the kind of the microorganism into which the gene is introduced or the gene to be introduced. In addition, enhancement of gene expression may be achieved by inducing endogenous gene expression by deleting a specific gene or region on the genome.

The method for enhancing gene expression is not limited to the above methods. In addition, the microorganism used in the present disclosure may have an enhanced gene expression as a result of natural mutation. Enhancement of gene expression can be confirmed by a known method such as Northern blotting and real-time PCR. Alternatively, enhancement of expression of the gene can be confirmed by actually culturing the microorganism and quantifying the protein encoded by the gene produced intracellularly or extracellularly (in the culture solution).

Adk catalyzes the reaction to form adenosine diphosphate and adenosine monophosphate from adenosine triphosphate and adenosine, and the reaction is reversible. Those skilled in the art can appropriately measure the Adk activity by a known technique, for example, by measuring the conversion to an adenosine monophosphate derivative under the presence of ATP and MgCl₂ (see, for example, Moffatt, B A et al. "Adenosine kinase of Arabidopsis. Kinetic properties and gene expression." Plant physiology vol. 124,4 (2000): 1775-85.).

Adk used in the present disclosure is not particularly limited, and is a polypeptide derived from eukaryotes such as a mammal, a plant and fungi, bacteria such as *actinomycetes,* archaea, or the corresponding polypeptide. Adk is preferably derived from a microorganism. Examples of Adk derived from a microorganism include a polypeptide comprising the amino acid sequence shown in SEQ ID NO: 1 or a variant sequence thereof derived from *Streptomyces coelicolor.*

MNRQPASPQERVHAVRIAVTGSIATDHLMTFPGRFSDQLVADQLHTVSLSFLVDQLDVRRGGVA
ANIAFGMGQLGTRPVLVGAAGSDFDEYRAWLDRHGVDTDSVRISETLHTARFVCTTDADHNQI
GSFYTGAMSEARLIELKTVADRVGGLDLVSIGADDPEAMLRHTEECRARSIPFAADFSQQIARMN
GDEIRILLDGATYLFSNEYEKGLIETKTGWSDAEILDRVGHRVTTLGARGVRIERAGGETIEVGVP
DEERKADPTGVGDAFRAGFLSGLAWGVSLERAAQVGCMLATLVIETVGTQEYQLRRGHFMERF
TKAYGDEAAGEVQAHLG (SEQ ID NO: 1)

An example of a gene encoding an Adk amino acid sequence is a gene comprising the nucleic acid shown in SEQ ID NO:2.

atgaatcggcaaccagcatctccgcaagaacgcgtgcatgcagtccgcattgcggtcaccggaagtatcgctaccgatcatctgatgac
ctttccaggacgcttctcggatcagttagtcgcggaccagcttcacaccgtgagcctgtcgtttctggtggatcagctcgatgttcgtcgc
ggaggcgtagccgccaacatcgcgttcggaatggggcaactgggtactcgtcctgtcctggttggtgccgctggcagtgacttcgacga
ataccgcgcatggttagatcgccacggggtcgatacggactcagttcgcatttcggaaaccctgcatactgcgcgtttcgtatgcaccac
cgatgctgatcacaaccagatcggctccttctatacaggtgccatgagcgaagcgcggctgattgagctgaaaacggttgcggatcgtgt
aggcggtttggacctggtgagcattggcgcggatgaccccgaagcgatgttacgtcatacggaggagtgtcgcgcgcgctcaattccgt
ttgcggcggactttagccagcaaattgcccgcatgaatggcgacgaaattcgcattcttctggatggcgccacatacctcttcagcaacg
agtacgagaagggcttgatcgaaacgaaaaccgggtggtccgacgccgaaatcttggatcgtgtgggtcatcgtgtgactacgctgggc
gctcgtggggttcgtatcgaacgtgcaggcggtgaaaccatcgaagttggtgtaccggatgaagaacgcaaagcggatccgaccggtgt
gggcgatgcgtttcgcgcaggctttctgtctggccttgcatggggtgtgagtctcgaacgcgctgcccaggttggttgcatgttagccac
cctggtcattgagactgtgggtacacaggagtatcagctgcgtcgcggccactttatggaacggtttacgaaagcctatggcgatgaagc
agcaggggaagtgcaagctcatctgggttaa (SEQ ID NO: 2)

Adk can be any polypeptide as long as it is functionally equivalent. Adk is, for example, a polypeptide according to the following (a)-(e):
(a) a polypeptide comprising the amino acid sequence shown in SEQ ID NO: 1;
(b) a polypeptide comprising an amino acid sequence comprising a substitution, a deletion, an insertion, or an addition of one to several amino acid residues in the amino acid sequence shown in SEQ ID NO: 1;
(c) a polypeptide comprising an amino acid sequence having 80% or more, 85% or more, 90% or more, 95% or more, 96% or more, 97% or more, 98% or more, or 99% or more sequence identity to the amino acid sequence shown in SEQ ID NO: 1;
(d) a polypeptide having an amino acid sequence encoded by a nucleotide sequence that hybridizes under stringent conditions with a nucleotide sequence encoding the amino acid sequence shown in SEQ ID NO: 1; or
(e) a polypeptide encoded by a nucleotide sequence having 70% or more, 75% or more, 80% or more, 85% or more, 90% or more, 95% or more, 96% or more, 97% or more, 98% or more, 99% or more sequence identity to a nucleotide sequence encoding the amino acid sequence shown in SEQ ID NO: 1.

In the present disclosure, "a substitution, a deletion, an insertion, or an addition of one to several amino acid residues" means substitution, deletion, insertion or addition of 1, 2, 3, 4, 5, 6, 7, 8, 9 or 10 amino acid residues, preferably means substitution, deletion, insertion or addition of 1, 2, 3, 4, 5 or 6 amino acid residues, and more preferably substitution, deletion, insertion or addition of 1, 2, 3 or 4 amino acid residues.

With respect to "hybridize under stringent conditions", hybridization as used herein can be performed according to a usual method described in, for example, Molecular Cloning, T. Maniatis et al., CSH Laboratory (1983). Examples of the "stringent conditions" include conditions under which a hybrid is formed in 6 × SSC (10 × SSC contains 1. 5M NaCl and 0. 15M trisodium citrate) and 50% formaldehyde at 45°C, and then washed with 2 × SSC at 50°C. (Molecular Biology, John Wiley & Sons, N. Y. (1989), 6.3.1-6.3.6), and conditions under which stringency equivalent thereto is achieved.

A microorganism used in the present disclosure is one having an ergothioneine biosynthetic gene. An ergothioneine biosynthetic gene is a gene encoding an enzyme involved in ergothioneine biosynthesis. The enzyme involved in ergothioneine biosynthesis and the ergothioneine biosynthetic gene are known, and the nucleotide sequences of some genes are also known. A person skilled in the art can appropriately select an ergothioneine biosynthetic gene to be expressed. For example, but not limited to, a microorganism that expresses an EGT biosynthetic gene by enhancing expression of any one or more of a gene corresponding to *egtA, egtB, egtC, egtD* or *egtE* of *Mycobacterium smegmatis,* either or both of a gene corresponding to *egtl* or *egt2* of *Schizosaccharomyces pombe,* either or both of a gene corresponding to *egt1* or *egt2* of *Neurospora crassa* or either or both of a gene corresponding to *eanA* or *eanB* of *Chlorobium limicola* are preferably used. With respect to the *Mycobacterium* ergothioneine biosynthetic genes, see F. P. Seebeck, J. Am. Chem. Soc., 2010, 132, 6632-6633. With respect to the ergothioneine biosynthetic genes of the genus *Schizosaccharomyces,* see T. Pluskal et al, PLOS ONE., 2014, 9(5), e97774. With respect to the ergothioneine biosynthetic genes of the genus Neurospora, see W. Hu et al., Org. Lett., 2014, 16, 5382-5385. With respect to the ergothioneine biosynthetic genes of the genus *Streptomyces,* see S. Nakajima et al., J. Biosci. Bioeng., 2015, 120, 294-298. With respect to the ergothioneine biosynthetic genes of the genus *Chlorobium,* see R. Burn, et al., Angew. Chem. Int. Edit., 2017, 56, 12508-12511. With respect to the ergothioneine biosynthetic genes in various microorganisms, see G. W. Jones et al, Gene, 2014, 549, 161-170.

A "corresponding gene" refers to a gene encoding a protein that has the same or similar function to a gene encoding a protein having a certain function. For example, a "corresponding gene" to an enzyme gene is a gene encoding an enzyme that catalyzes the same or similar reaction to the enzyme to a gene encoding an enzyme that catalyzes a certain reaction. A certain gene and the corresponding gene may have the same nucleotide sequence or different nucleotide sequences. For example, nucleotide sequence identity between a certain gene and the corresponding gene may have 50% or more, 70% or more, 80% or more, or 90% or more. Furthermore, an enzyme protein encoded by a certain gene and an enzyme protein encoded by a gene corresponding to the certain gene may have the same amino acid sequence or different amino acid sequences. For example, the amino acid sequence identity between the enzyme protein encoded by a gene and the enzyme protein encoded by the gene corresponding to the certain gene may be 50% or more, 70% or more, 80% or more, or 90% or more.

A microorganism having a gene for biosynthesis of ergothioneine or a related substance thereof used in the present disclosure may be any microorganism. A microorganism capable of producing ergothioneine or a related substance thereof is, for example, a microorganism belonging to the family *Enterobacteriaceae* or to the order *Actinomycetes.* A microorganism belonging to the *Enterobacteriaceae* family includes, but is not limited to, a microorganism belonging to the genera *Escherichia, Enterobacter, Pantoea, Klebsiella,* and *Salmonella.* A particularly preferred microorganism belonging to the family *Enterobacteriaceae* includes those belonging to the genus *Escherichia,* such as *Escherichia coli,* and those belonging to the genus *Pantoea,* such as *Pantoea ananatis.* A microorganism belonging to the order *Actinomycetes* is not particularly limited, but is a microorganism belonging to the genera *Streptomyces, Corynebacterium,* and *Mycobacterium.* A particularly preferred microorganism belonging to the family *Streptomyces* includes *Streptomyces lividans, Streptomyces coelicolor, Streptomyces avermitilis*, *Streptomyces griseus, Streptomyces albus,* and *Streptomyces albulus.*

Cultivation of a microorganism in the method for the production of the present disclosure can be performed by a usual method. Various conditions such as media composition, culture temperature, culture time, pH and aeration conditions can be selected according to the type of microorganism. Ergothioneine can be produced by culturing the above microorganism in a known medium, and the medium may contain a compound that contributes to ergothioneine biosynthesis, such as histidine, methionine, or cystine or precursor thereof. In one embodiment, LB medium, 2xYT medium, NZY medium, M9 medium, SOC medium, YPD medium, or the like can be used. Ergothioneine can be produced using the medium described above, but the medium is not limited to these medium. In addition, the produced ergothioneine or a related substance thereof may be accumulated within the cells, or may be secreted or accumulated outside the cells (in the culture solution).

Ergothioneine or a related substance thereof within the cells or released from the cells can be recovered by a known method. For example, the culture is subjected to solid-liquid separation such as centrifugation or filtration, and an extract containing ergothioneine or a related substance thereof can be obtained from the cells by solvent extraction, hot water extraction, crushing treatment, or the like. The extract or culture supernatant containing ergothioneine or a related substance thereof can be subjected to known chromatography such as ion exchange chromatography, hydrophobic chromatography, gel filtration chromatography to obtain ergothioneine or a related substance thereof.

In a second aspect, the present disclosure relates to a microorganism having an ergothioneine biosynthetic gene, wherein the microorganism has an increased Adk activity. The ability to produce ergothioneine or a related substance thereof can be increased by using the microorganism of the present disclosure.

As used herein, "about" means a range of ±10%, preferably ±5%.

The present disclosure will be described in more detail and specifically below by showing examples, but the examples are not intended to limit the scope of the present disclosure. Unless otherwise specified, experiments were carried out using the methods described in the standard protocols for molecular biology and applied microbiology, or the modified or altered methods. Also, % represents w/v % unless otherwise specified.

### EXAMPLES

### Example 1

### Construction of adk co-expression vector

For the purpose of highly expressing an EGT biosynthetic enzyme and *adk*, the following *adk* co-expression vector was constructed. Adk (SCO2158: SEQ ID NO: 1) derived from *Streptomyces (Streptomyces coelicolor)* was used as an adenosine kinase. A codon-optimized *adk* gene (SEQ ID NO: 2) was artificially synthesized for expression in *E. coli. Adk* was amplified using the vector containing *adk* as a template and the primers listed in Table 1. *OmpA* promoter was used as the *adk* promoter. *OmpA* promoter was amplified using the *E. coli* genomic DNA as a template and the primers listed in Table 1.

**[Table 1]**

| Primer | Sequence |
|---|---|
| Sc.adk-Fw | 5'-AACGAGGCGCAAAAAATGAATCGGCAACCAGCATCTCC-3' (SEQ ID NO: 3) |
| Sc.adk-Rv | 5'-AGCTACTAGTCTCGATTAACCCAGATGAGCTTGCACTTCCC-3' (SEQ ID NO: 4) |
| OmpAp -Fw | 5'- CGGCCGCTCATATGCTCGAGGTGACTAATTTTCCTTGCGGAGGC TTG-3' (SEQ ID NO: 5) |
| OmpAp -Rv | 5'-TTTTTGCGCCTCGTTATCATCCAAAATAC-3' (SEQ ID NO: 6) |

An expression vector was constructed using pACG-EGT as described in WO2019/163767. The *ompA* promoter and *adk* PCR product were ligated to the XhoI site of pACG-EGT using gibson assembly master mix (NEW ENGLAND BioLabs) to construct pACG-EGT_*adk* as an *adk* co-expressing EGT production vector. Gibson Assembly was performed according to the protocol provided by NEW ENGLAND BioLabs.

BW25113 strain (NBRC 103404) was used as the host strain of *E. coli.*

The BW25113 strain was used for a transformation with pACG-EGT_*adk* to obtain an *adk* co-expressing EGT producing strain (BW25113/pACG-EGT_*adk*).

### Example 2

### EGT production test in E. coli

The EGT-producing strain BW25113/pACG-EGT_*adk* constructed in Example 1 was used to conduct an EGT fermentation production test. BW25113/pACG-EGT, in which pACG-EGT was introduced into the BW25113 strain, was used as a control strain. Each glycerol stock of BW25113/pACG-EGT and BW25113/pACG-EGT_*adk* was added to a test tube containing tetracycline (final concentration: 10 µg/mL) in 3mL of LB medium (10g of polypeptone, 5g of yeast extract, 10g of NaCl/1L of medium), and cultured with shaking at 30°C and 200 rpm for 15 to 18 hours by the stationary phase to obtain a preculture solution. The preculture solution was added to 5mL of 2×YT medium (polypeptone 16 g, yeast extract 10g, NaCl 5g, glucose 5%, L-histidine 5mM, L-cystine 2.5mM, L-methionine 5mM, tetracycline 10 µg/mL/1L of medium) in a Φ24 mm test tube, so as to be OD600=0.5, and the main culture was performed by shaking at 30°C and 300 rpm for 96 hours.

EGT was quantified as follows. During the main culture, 1mL of the culture solution was collected at 24, 48, 72, and 96 hours after the initiation of culture, and the culture samples were evaluated. The cell turbidity (OD600) of the sampled culture solution was measured, and the culture solution was centrifuged at 14000 rpm for 10 minutes to separate the extracellular culture solution and the precipitate (bacterial cells). The EGT content in the separated extracellular culture medium (extracellular sample) was measured by HPLC. The HPLC measurement conditions are as shown in Table 2 below, and quantification was performed based on the EGT peak occurring at a retention time of about 10 minutes.

**[Table 2]**

| | |
|---|---|
| Column | Inertsil HILIC (5µM; 4.6ϕmm×250mm) GL Sciences |
| Solvent | 100mM ammonium acetate/acetonitrile/ Isopropanol/water (2:82:6:10) |
| Gradient conditions | Isocratic |
| Detection wavelength | 265 nm |
| Flow rate | 1mL/min |
| Column temperature | 40°C |

As a result of the culture test, the EGT production of the control strain BW25113/pACG-EGT and the *adk* co-expressing strain BW25113/pACG-EGT_*adk* were confirmed after 72 hours of culture. The EGT productivity in the *adk* co-expressing strain was improved by about 3.0-fold compared to the control strain, and the EGT productivity was surprisingly remarkably improved by the co-expression of *adk* (FIG. 1).

### INDUSTRIAL APPLICABILITY

The method for producing ergothioneine, a related substance thereof, or a mixture thereof according to the present invention can be used to produce ergothioneine having various physiological activities including a strong antioxidant effect inexpensively and in a large amount.

### SEQUENCE LISTING FREE TEXT

SEQ ID NO: 1 is the amino acid sequence of the *adk* gene (SCO2158) derived from *Streptomyces coelicolor.*
SEQ ID NO: 2 is the nucleotide sequence of the *adk* gene codon-optimized for expression in A. *coli.*
SEQ ID NO:3 is the nucleotide sequence of the forward primer for amplifying the *adk* gene shown in SEQ ID NO:2.
SEQ ID NO: 4 is the base sequence of the reverse primer for amplifying the *adk* gene shown in SEQ ID NO: 2.
SEQ ID NO: 5 is the base sequence of a forward primer for amplifying the promoter of the *ompA* gene derived from *E. coli.*
SEQ ID NO: 6 is the base sequence of a reverse primer for amplifying the promoter of the *ompA* gene derived from *E. coli.*

## Claims

1. A method for producing ergothioneine or a related substance thereof, or a mixture thereof, comprising:
culturing in a medium a microorganism having an ergothioneine biosynthetic gene, and collecting ergothioneine or a related substance thereof, or a mixture thereof from the medium obtained by the culture or the bacterial cells,
wherein the microorganism is a strain modified to increase adenosine kinase (Adk) activity.

2. The method of claim 1, wherein the Adk activity is increased by enhancing an expression of a gene encoding Adk.

3. The method of claim 1 or 2, wherein the microorganism belongs to the family *Enterobacteriaceae* or to the order *Actinomycetes.*

4. A microorganism having an ergothioneine biosynthetic gene, wherein the microorganism has an increased Adk activity.

5. The microorganism of claim 4, wherein the Adk activity is increased by enhancing an expression of a gene encoding Adk.

6. The microorganism of claim 4 or 5, wherein the microorganism belongs to the family *Enterobacteriaceae* or to the order *Actinomycetes.*
